# EUROPEAN PATENT SPECIFICATION

(11) **EP 3 030 890 B1**
(45) Date of publication and mention of the grant of the patent: **01.05.2019**
(21) Application number: 14834533.3
(22) Date of filing: 11.08.2014
(51) Int. Cl.: G01N 27/26, A61B 5/1473, G06F 19/00, A61B 5/145

(54) **USING KINETIC CYCLIC VOLTAMMETRY TO EVALUATE ANALYTE KINETICS AND CONCENTRATIONS**
VERWENDUNG VON KINETISCHER VOLTAMMETRIE ZUR BEURTEILUNG VON ANALYTKINETIK UND -KONZENTRATIONEN
UTILISATION DE LA VOLTAMPÉROMÉTRIE CYCLIQUE CINÉTIQUE POUR ÉVALUER LES CINÉTIQUES ET LES CONCENTRATIONS D'ANALYTES

(30) Priority: 09.08.2013 US 201361864116 P
(43) Date of publication of application: 15.06.2016
(73) Proprietor: Mayo Foundation for Medical Education and Research, Rochester, MN 55905 (US)
(72) Inventor: LEE, Kendall H., Rochester, Minnesota 55902 (US); JANG, Dong-Pyo, Sungnam city Gyeonggi-do 463-706 (KR)
(74) Representative: Kunz, Herbert
(86) International application number: PCT/US2014/050550
(87) International publication number: WO 2015/021470

(56) References cited:
- US-A- 5 650 061
- US-A1- 2004 108 223
- US-A1- 2008 179 197
- US-A1- 2010 032 316
- US-A1- 2012 165 634
- US-A1- 2013 023 745
- GORDON N. ECCLES ET AL: "Pulse cyclic voltammetry. II. Flowing solutions", CANADIAN JOURNAL OF CHEMISTRY, vol. 65, no. 8, 1 August 1987 (1987-08-01) , pages 1795-1799, XP55343831, CA ISSN: 0008-4042, DOI: 10.1139/v87-301

## Description

### TECHNICAL FIELD

This document relates to materials and methods for using kinetic cyclic voltammetry to measure analyte levels and assess analyte kinetics.

### BACKGROUND

Electrochemical techniques can be useful for monitoring the release and/or uptake of various metabolites *in vitro* and *in vivo,* based on voltage-dependent oxidation and reduction processes. For example, cyclic voltammetry is a type of potentiodynamic electrochemical measurement that can be used to evaluate the electrochemical properties of an analyte in solution. Cyclic voltammetry (CV) generally involves ramping the potential of a working electrode linearly versus time, like linear sweep voltammetry. Unlike linear sweep voltammetry, which ends when it reaches a set potential, the working electrode's potential ramp in cyclic voltammetry is inverted when it reaches a set potential. The inversion can happen multiple times during a single evaluation. The current at the working electrode can be plotted versus the applied voltage to give the cyclic voltammogram trace.

US 2013/0023745 A1 describes methods and materials for using paired pulse voltammetry to discriminate analytes on the basis of their adsorption characteristics to an electrode.

US 2004/0108223 A1 describes a method of signal processing for voltammetry based on the customizing of a univariate mathematical model.

Eccles GN et al. describe Fourier-smoothed pulse cyclic chronovoltammograms in the Canadian Journal of Chemistry, vol. 65, no. 8, 1 August 1987, on pages 1795-1799.

### SUMMARY

The present invention is defined by the independent claims. The dependent claims depict preferred embodiments of the invention.

The use of electrochemistry [e.g., fast scan cyclic voltammetry (FSCV)] to evaluate analytes presents challenges that can include difficulty differentiating multiple complex analytes, and the inability to measure absolute analyte amounts. The present document is based in part on the development of kinetic cyclic voltammetry (KCV), an electrochemical technique in which multi pulse cyclic voltammetry per scan can be done at various voltages. KCV can be used to generate kinetic maps (K-maps) that show specific kinetic characteristics of particular analytes. Unlike conventional cyclic voltammetry, KCV also can be used to generate concentration maps (A-maps) for such analytes. The K-maps and A-maps can be obtained without background subtraction. Thus, KCV makes absolute quantification of analytes possible. Moreover, this technology can be incorporated into a smart neuromodulation system, because signal analysis can occur in real time.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a diagram of an exemplary kinetic cyclic voltammetry (KCV) waveform.
FIG. 2 is a schematic showing a KCV waveform with dopamine (DA) injection (top), as well as background current waveforms before and after DA injection (middle), and a resulting background-subtracted cyclic voltammogram.
FIG. 3 is a diagram and equations for a kinetic model for adsorption and desorption of DA and dopamine quinone (DOQ).
FIG. 4 is a diagram depicting an exemplary kinetic fast scan cyclic voltammetry waveform (top and middle), and a pair of equations for determining detection values of DA and DOQ in the first pulse (1) (bottom).
FIG. 5 shows a series of equations for determining detection values of DA and DOQ in the second (2) and third (3) pulses shown in the middle portion of FIG. 4.
FIG. 6 shows a pair of equations for determining detection values for DA and DOQ in a particular pulse (m) (top), as well as a series of equations for determining the difference in detection values for DA and DOQ in consecutive pulses (m+1 and m) (middle and bottom).
FIG. 7 is a graph plotting a background subtracted cyclic voltammetry waveform.
FIG. 8 is a graph plotting an exponential curve fitting the difference between two consecutive pulses (left), and a pseudo *k* value color map and *A* value color map (right) indicating kinetic characteristics and concentration, respectively.
FIG. 9 is a FSCV pseudo color map (top) showing DA oxidation and reduction, as indicated, and a FSCV waveform (bottom) for the point along the pseudo color map indicated by the dashed line.
FIG. 10 shows a K-map and corresponding pseudo *k* value color map for DA (left), and an A-map and corresponding pseudo *A* value color map for DA (right).
FIG. 11 is a series of A-maps (top) and K-maps (bottom) for increasing concentrations of DA, as indicated.
FIG. 12 is a graph plotting concentration vs. A value for DA.
FIG. 13 is a series of images showing different FSCV, *A* map, and *k* map patterns for DA (left), ascorbic acid (center), and 5-hydroxytryptophan (right), as indicated.
FIG. 14 is a series of images showing FSCV, *A* map, and *k* map patterns for adenosine, as indicated.
FIG. 15 is a KCV waveform showing what happens when kinetic analysis is applied to CV for DA + Tris buffer, without background subtraction.
FIG. 16 is a series of images showing *A* maps and *K* maps for increasing concentrations of DA in Tris buffer, as indicated, with kinetic information estimated with original CV (without background subtraction).
FIG. 17 is a block diagram of computing devices that may be used to implement the systems and methods described in this document, as either a client or as a server or plurality of servers.

### DETAILED DESCRIPTION

"Voltammetry" refers to electroanalytical methods that can be used in analytical chemistry to give information about an analyte, obtained by measuring current as the potential is varied. In FSCV, for example, a voltage sufficient to oxidize one or more analytes (e.g., a monoamine) is applied to a carbon fiber microelectrode. The oxidation process results in current flow at the electrode surface, and the amount of current is converted into concentration of monoamine in the vicinity of the electrode tip by means of calibration in a flow injection system. Plotting the current against time can provide information as to how various parameters and agents alter the dynamics of the analyte(s) released near the surface of the electrode.

The present document is based in part on the development of KCV, a technique in which multi pulse cyclic voltammetry per scan (e.g., 10 triangle pulses per scan) can be done at various voltages (e.g., from -0.4 volt to +1.0 volt and back down to -0.4 volt, at 1000 volts per second). As described herein, KCV methods can be used to obtain differential information related to adsorption characteristics of multiple analytes or metabolites within a sample or testing environment (e.g., within the brain of a mammal) by controlling the electrical potential repetition cycle time using a device or system such as a wireless instantaneous neurotransmitter concentration system (WINCS). Using KCV as described herein can allow for improved estimation of specific analyte and metabolite concentrations in complex electrochemical environments.

This document provides methods and materials involved in detecting, differentiating, and quantifying analytes detected using KCV. For example, this document provides materials and methods for using KCV to discriminate analytes based on their adsorption characteristics to an electrode (e.g., a carbon fiber electrode). As described herein, KCV can be used to assess a concentration of an analyte present within a tissue *in vivo* or *in vitro.* For example, the methods and materials provided herein can be used to assess the concentration of an analyte (e.g., a chemical such as a neurochemical or an ion) within brain tissue. In some cases, the methods and materials provided herein can be used to assess the concentration of an analyte during deep brain stimulation. Examples of analytes that can be detected using the methods and materials provided herein include, without limitation, ions such as calcium, magnesium, sodium, potassium, protons (pH), iron, copper, chromium, lead, mercury, cobalt, gold, lithium, cesium, barium, zinc, chloride, bicarbonate, phosphate, bromide, iodide, sulfide, oxide, sulfide, and fluoride, chemicals such as dopamine, serotonin, adenosine, adenine mono- or tri-phosphate, norepinephrine, GABA, histamine, acetylcholine, glutamate, aspartate, epinephrine, nitric oxide, glycine, trace amines (e.g., tryptamine, phenylethylamine, tyramine, and octopamine), and amino acid-based neuropeptides (e.g., endorphins, enkephalins, and vasopressin). In some embodiments, for example, KCV can be used to assess the concentration of one or more chemicals (e.g., dopamine or adenosine).

Any appropriate device with the ability to do variable timing of voltammetry can be used to obtain KCV data by applying a waveform (e.g., a binary waveform) to a tissue being assessed. For example, a WINCS device (see, e.g., PCT Publication No. WO2011/028608) can be used to obtain voltammetric data pertaining to a tissue being assessed. Other suitable devices include, for example, The Universal Electrochemistry Instrument (UEI; Department of Chemistry Electronic Shop, University of North Carolina) and Pinnacle's Fast Scan Cyclic Voltammetry system (Pinnacle Technology, Inc., Lawrence, KS). In some cases, a voltammetry device can include one or more electrodes or sensors to detect one or more analytes. In some cases, a single electrode or sensor can be used to detect a single chemical. For example, a voltammetry device can include a first electrode designed to detect dopamine and a second electrode designed to detect glutamate. Another design uses a singular sensing electrode to detect different chemicals by applying different voltage ramps at slightly different times.

In some cases, a device (e.g., a WINCS device) can be implanted within a patient. For example, a WINCS device can be implanted within a patient's skull. In some cases, a device can include a single electrode for KCV that contains two independent areas of active electrode such as pyrolytic carbon or carbon fiber. Then by varying the KCV impressed voltage, signals representing different neurochemicals can be determined. If the active electrode areas are too close together, which would cause interference, the signals can be multiplexed such that the signal is detected quasi simultaneously by measuring one chemical directly after a preceding chemical. The device can also be used to determine the effect of stimulation. For example, stimulation of two different areas of the brain can produce a release of different neurochemicals (such as histamine, adenosine, glutamate, and dopamine). The ratio or absolute amount changes of the released neurochemicals can provide a physiologic effect of interest, such as creating long-term memory in patients with short-term memory loss.

In some cases, one or more chemicals (e.g., dopamine, adenosine, serotonin, and norepinephrine) can be detected using KCV with various scan waveforms applied to one or more electrodes or sensors. The scan waveforms can be varied by manipulating physical aspects of the waveforms such as the peak voltage, voltage ramp, and repetition time.

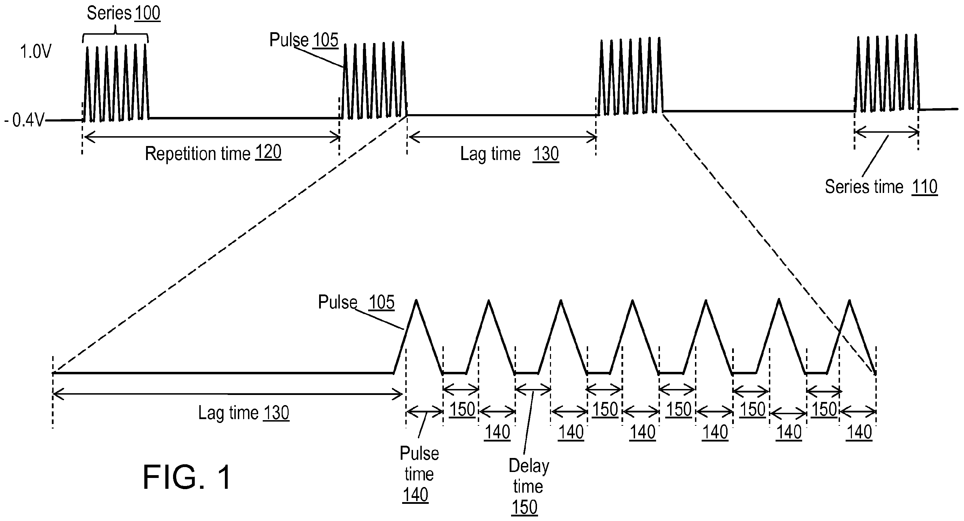

When using KCV to assess the concentration of one or more particular analytes, a series of pulses is rapidly generated, and the series is repeated an appropriate number of times. With reference to FIG. 1, for example, a pulse series 100 can be generated from two to 20 times (e.g., two, three, four, five, six, seven, eight, nine, 10, 11, 12, 13, 14, 15, 16, 17, 18, 19, or 20 times), with each series including from three to 20 individual pulses (e.g., three, four, five, six, seven, eight, nine, 10, 11, 12, 13, 14, 15, 16, 17, 18, 19, or 20 pulses; *see,* individual pulse 105 in FIG. 1). Any appropriate series time 110, repetition time 120, and lag time 130 can be used. For example, series times ranging from about 1 millisecond to about 300 milliseconds (e.g., from about 1 millisecond to about 250 milliseconds, from about 1 millisecond to about 200 milliseconds, from about 1 millisecond to about 150 milliseconds, from about 1 millisecond to about 100 milliseconds, from about 3 milliseconds to about 200 milliseconds, from about 3 milliseconds to about 100 milliseconds, from about 3 milliseconds to about 50 milliseconds, from about 20 milliseconds to about 200 milliseconds, from about 20 milliseconds to about 100 milliseconds, from about 20 milliseconds to about 50 milliseconds, from about 30 milliseconds to about 300 milliseconds, from about 30 milliseconds to about 200 milliseconds, from about 30 milliseconds to about 100 milliseconds, or from about 30 milliseconds to about 50 milliseconds) can be used. In some embodiments, repetition times ranging from about 10 milliseconds to about 10 seconds (e.g., from about 10 milliseconds to about 5 seconds, from about 10 milliseconds to about 2 seconds, from about 10 milliseconds to about 1 second, from about 10 milliseconds to about 750 milliseconds, from about 10 milliseconds to about 500 milliseconds, from about 10 milliseconds to about 250 milliseconds, from about 10 milliseconds to about 100 milliseconds, from about 50 milliseconds to about 150 milliseconds, from about 40 milliseconds to about 1 second, from about 50 milliseconds to about 1 second, or from about 75 milliseconds to about 1 second) can be used. In some embodiments, lag times ranging from about 1 millisecond to about 700 milliseconds (e.g., from about 1 millisecond to about 500 milliseconds, from about 1 millisecond to about 300 milliseconds, from about 1 millisecond to about 200 milliseconds, from about 1 millisecond to about 100 milliseconds, from about 7 milliseconds to about 500 milliseconds, from about 7 milliseconds to about 200 milliseconds, from about 7 milliseconds to about 100 milliseconds, from about 10 milliseconds to about 700 milliseconds, from about 30 milliseconds to about 700 milliseconds, from about 50 milliseconds to about 700 milliseconds, from about 50 milliseconds to about 300 milliseconds, from about 50 milliseconds to about 150 milliseconds, or from about 10 milliseconds to about 100 milliseconds) can be used.

In some cases, longer repetition and lag times than those set forth above can be used. For example, repetition and lag times of minutes or hours (e.g., about 1 minute, about 5 minutes, about 10 minutes, about 15 minutes, about 20 minutes, about 30 minutes, about 40 minutes, about 45 minutes, about 60 minutes, about 90 minutes, about 2 hours, about 2.5 hours, about 3 hours, or more than 3 hours) can be used.

Further, any appropriate pulse time 140 and delay time 150 (FIG. 1) can be used. For example, pulse times ranging from 0.15 millisecond to about 20 milliseconds (e.g., (e.g., from about 0.15 millisecond to about 15 milliseconds, from about 0.15 millisecond to about 10 milliseconds, from about 0.15 millisecond to about 5 milliseconds, from about 0.15 millisecond to about 2 milliseconds, from about 1 millisecond to about 20 milliseconds, from about 1 millisecond to about 10 milliseconds, from about 1 millisecond to about 5 milliseconds, from about 1 millisecond to about 3 milliseconds, from about 0.5 millisecond to about 20 milliseconds, or from about 1.5 milliseconds to about 20 milliseconds), and delay times ranging from about 0.2 millisecond to about 20 milliseconds (e.g., from about 0.2 millisecond to about 15 milliseconds, from about 0.2 millisecond to about 10 milliseconds, from about 0.2 millisecond to about 5 milliseconds, from about 0.2 millisecond to about 3 milliseconds, from about 1 millisecond to about 20 milliseconds, from about 1 millisecond to about 10 milliseconds, from about 1 millisecond to about 5 milliseconds, from about 1 millisecond to about 3 milliseconds, from about 0.5 millisecond to about 20 milliseconds, or from about 1.5 milliseconds to about 20 milliseconds) can be used.

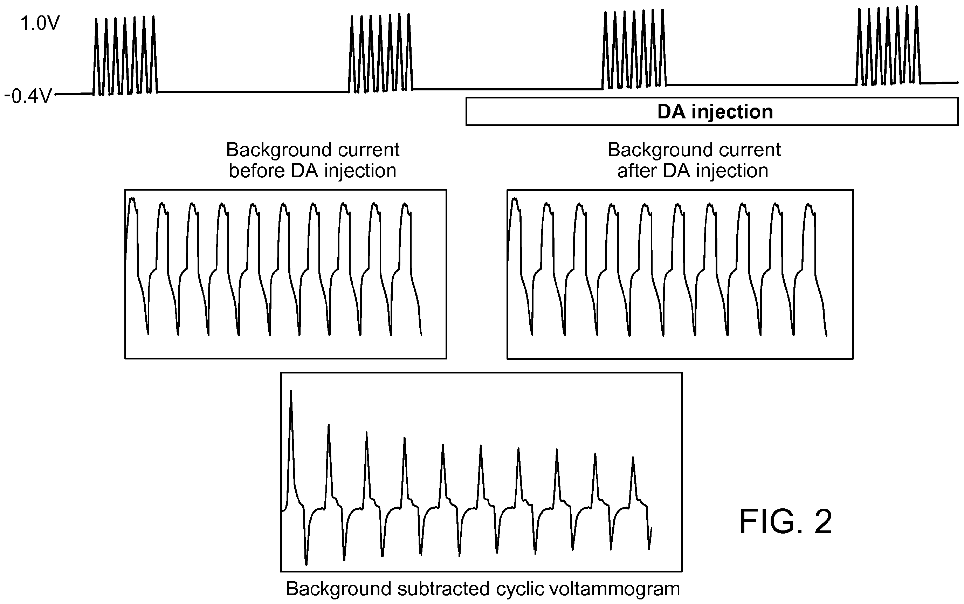

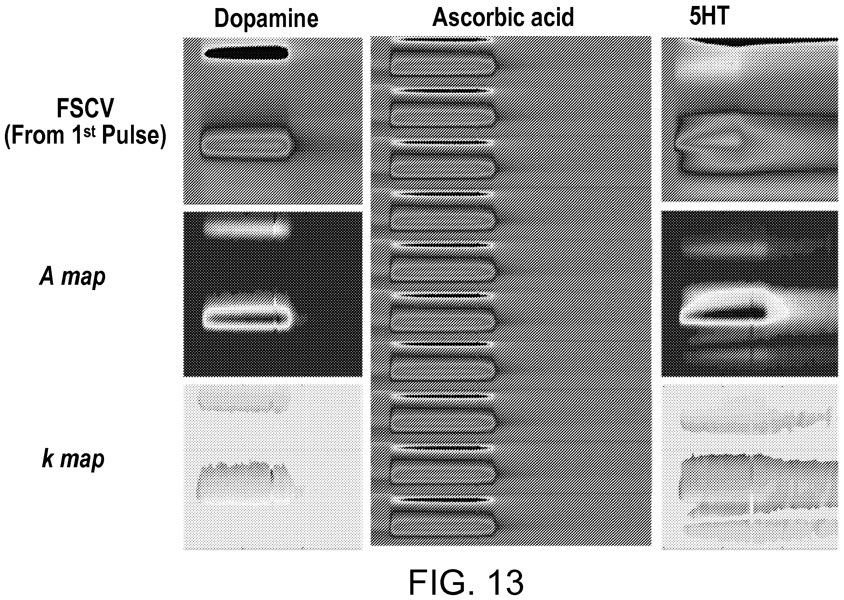

Figures 2-16 demonstrate the use of KCV to detect and quantify dopamine (DA) and dopamine quinone (DOQ). For example, the top panel of Figure 2 shows a KCV waveform for DA. The middle panel shows the background current waveforms before (left) and after (right) DA injection, and the resulting background-subtracted cyclic voltammogram is shown at the bottom.

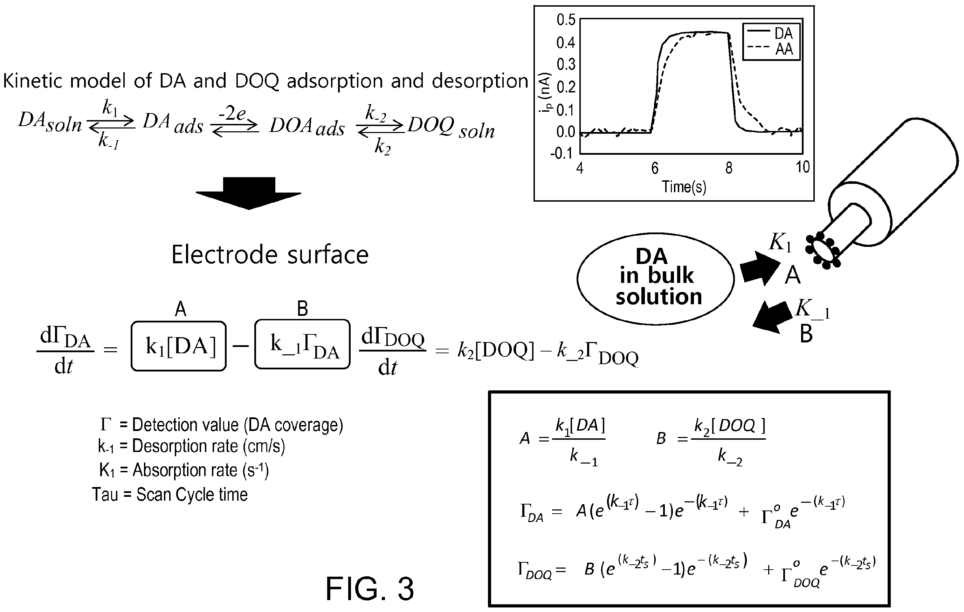

An explanation for the changing pattern is depicted in Figure 3, which depicts a kinetic model for adsorption and desorption of DA and DOQ:

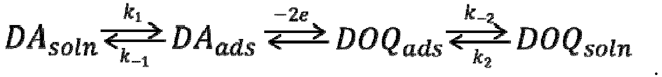

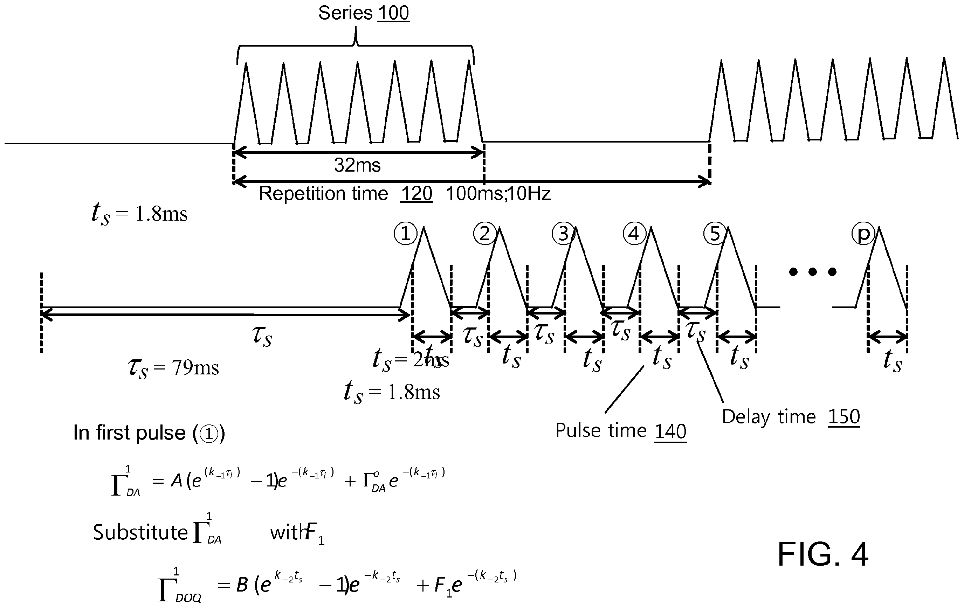

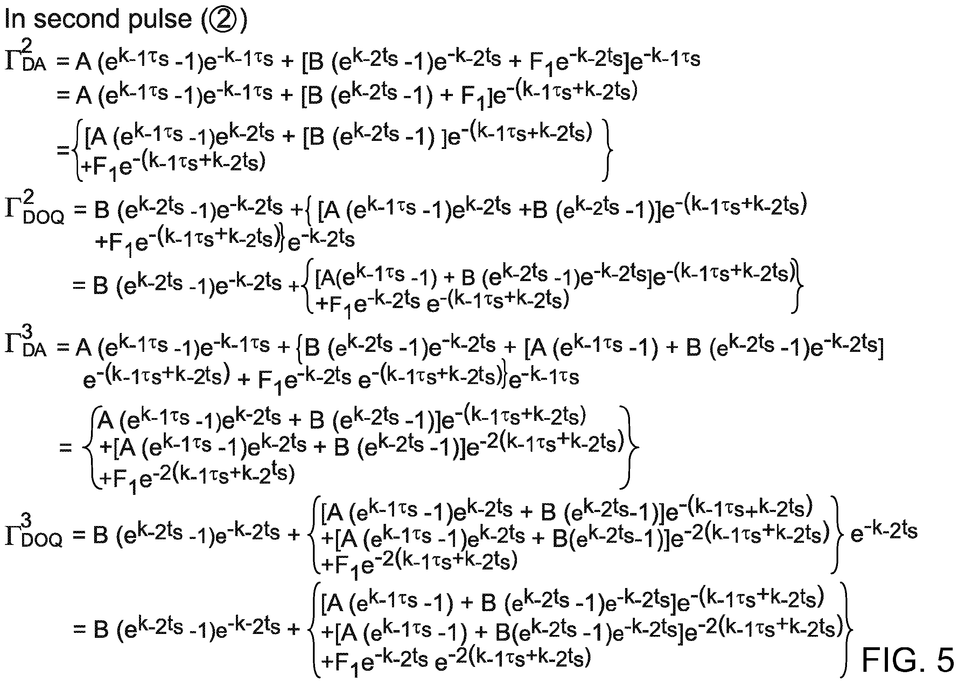

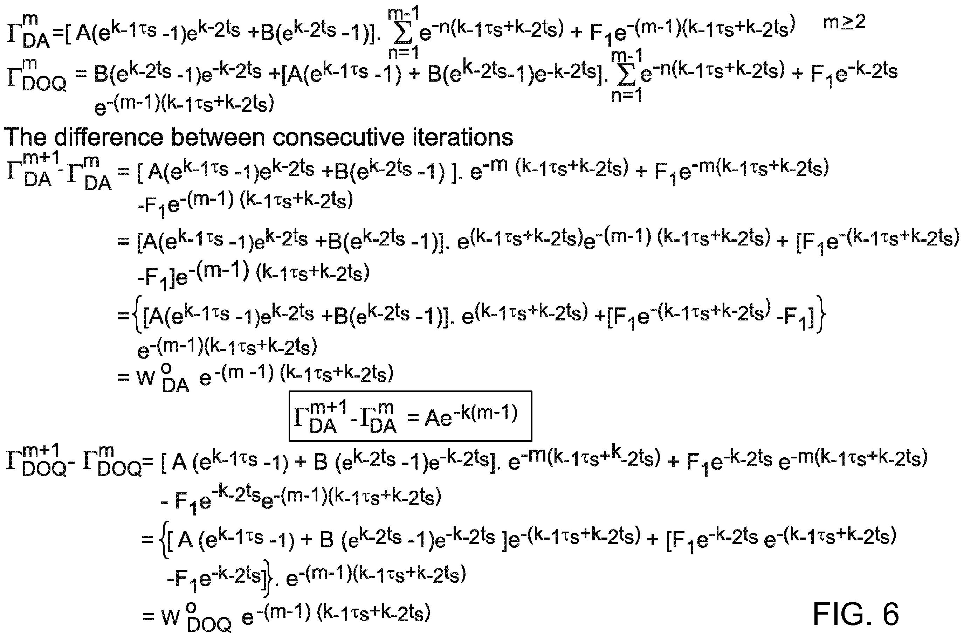

An exemplary KCV waveform is shown at the top of Figure 4, with pulse series 100 shown in expanded form in the middle panel. In this example, the repetition time 120 is 100 ms, the lag time (τ*ₗ*) is 79 ms, the pulse time (*tₛ*) is 1.8 ms, and the delay time (τₛ) is 2 ms. The bottom panel of Figure 4 shows equations for determining detection values for DA (Γ¹_{DA}) and DOQ (Γ¹_{DOQ}) in the first pulse. Equations for determining DA and DOQ detection values in the second, third, and subsequent pulses are shown in Figures 5 and 6. Figure 6 also provides a series of equations for determining the difference in detection values for DA and DOQ in consecutive pulses (m and m+1).

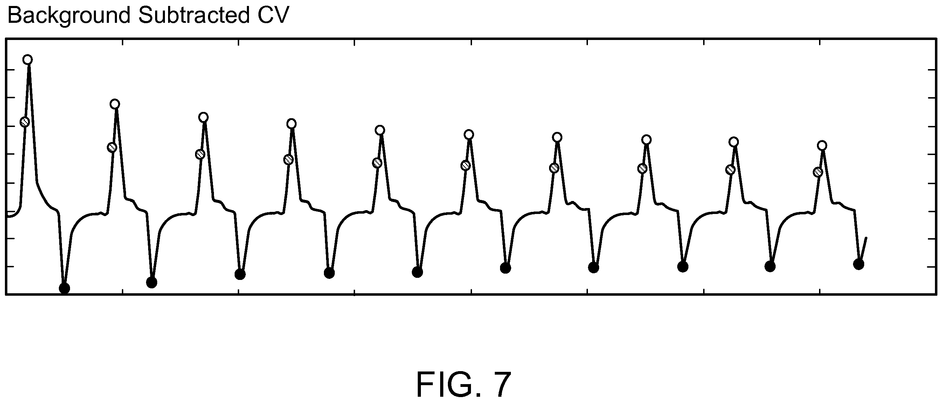

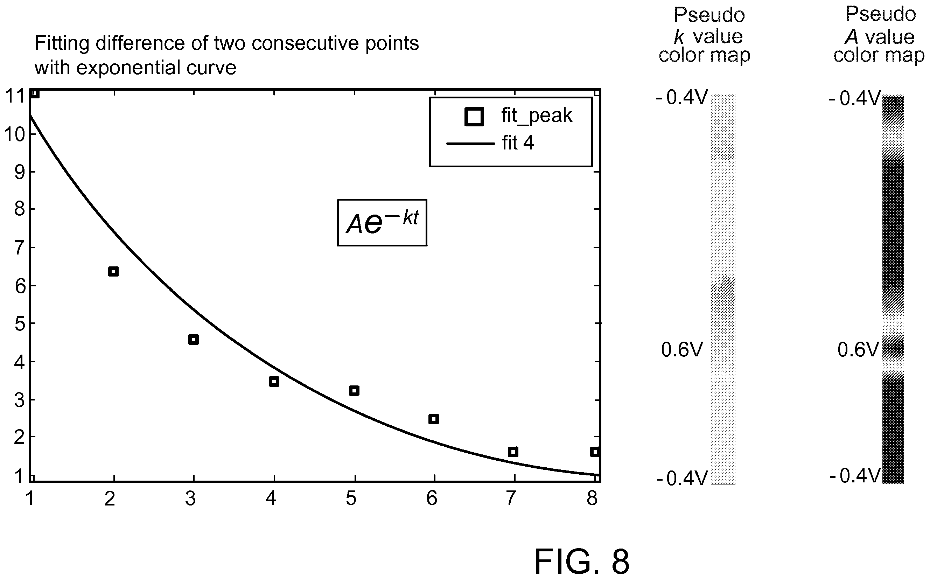

A background subtracted CV waveform is shown in Figure 7. The difference between two consecutive points can be fit to an exponential curve, as depicted in Figure 8 (left panel).

The right panels of Figure 8 show a pseudo *k* value color map indicating the kinetic characteristics of the analyte, and a pseudo *A* value color map indicating concentration.

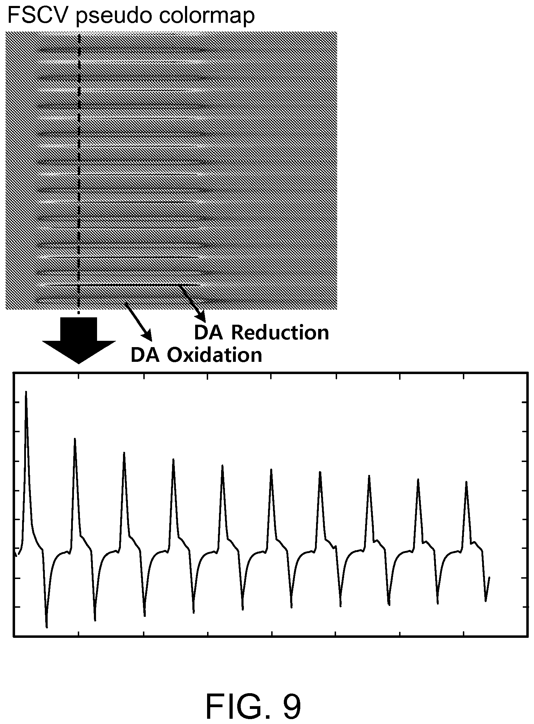

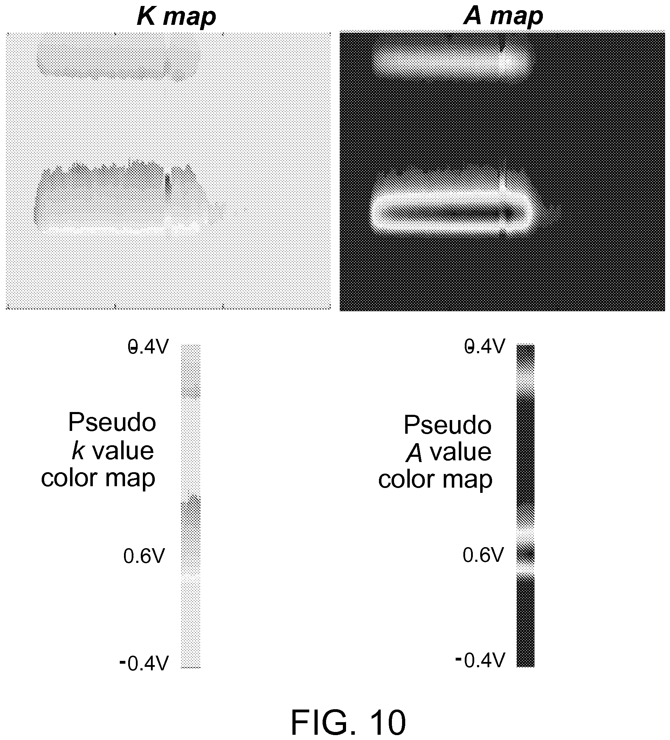

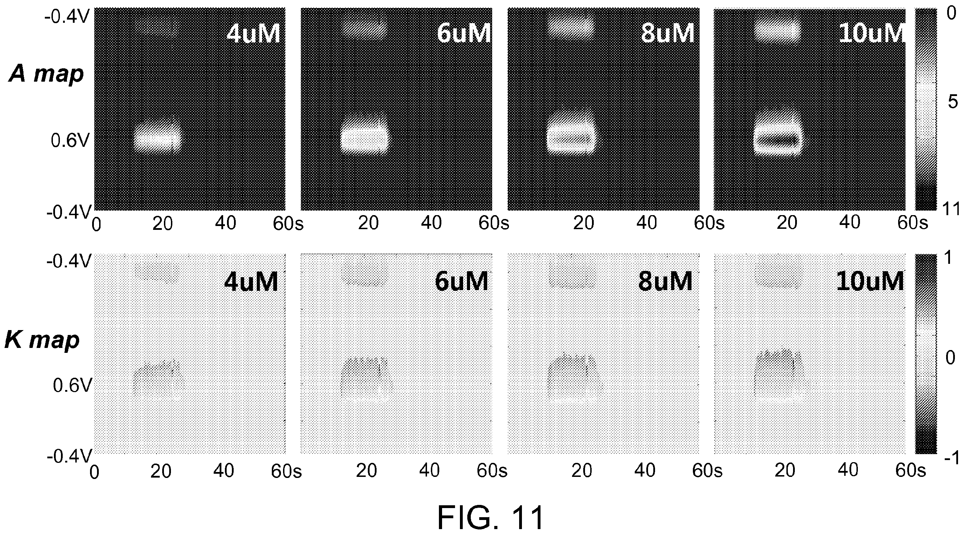

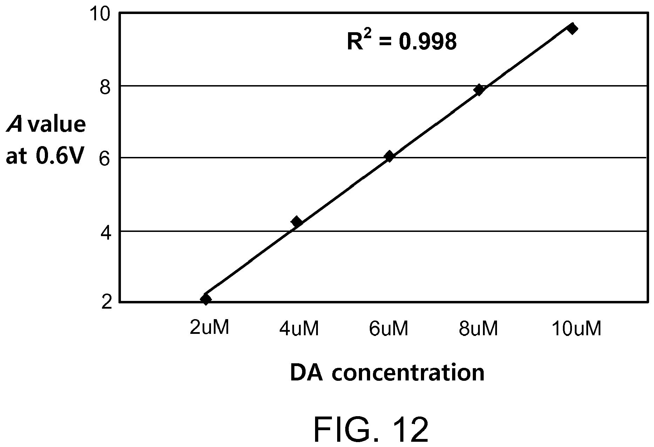

Oxidation and reduction of DA are indicated in the FSCV pseudo color map shown at the top of Figure 9, with a corresponding FSCV waveform shown at the bottom. A *K* map and corresponding pseudo *k* value color map for DA are presented in the left portion of Figure 10, and an *A* map and corresponding pseudo *A* value color map for DA are presented in the right portion. Increasing the concentration of DA leads to increased intensity in the *A* maps and *K* maps, as depicted in Figure 11. The linearity of the *A* value at 0.6 V vs. DA concentration is indicated in the graph shown in Figure 12 (R² = 0.998).

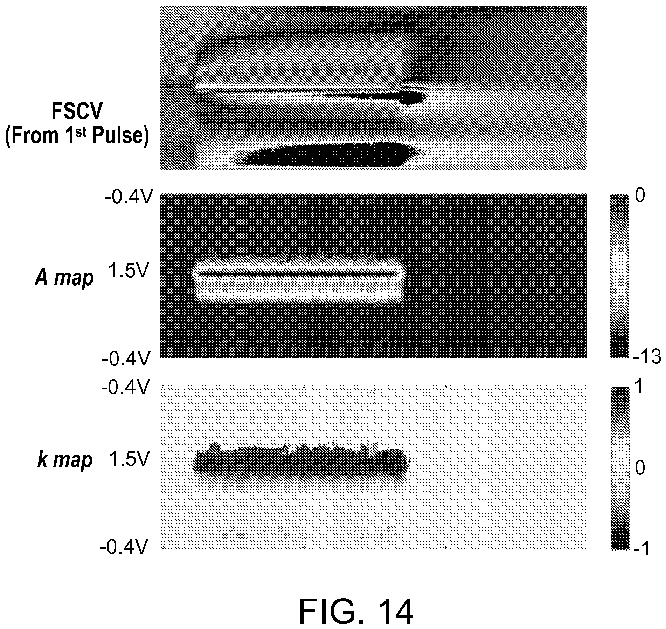

The *A* map, *k* map, and FSCV patterns vary based on the metabolite under examination. A series of images showing different FSCV, *A* map, and *k* map patterns for DA (left), ascorbic acid (center), and 5-hydroxytryptophan (right). Images for adenosine are shown in Figure 14.

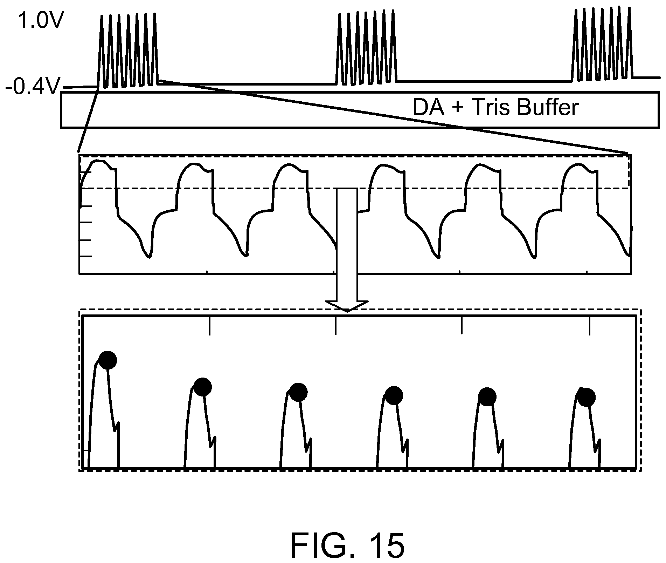

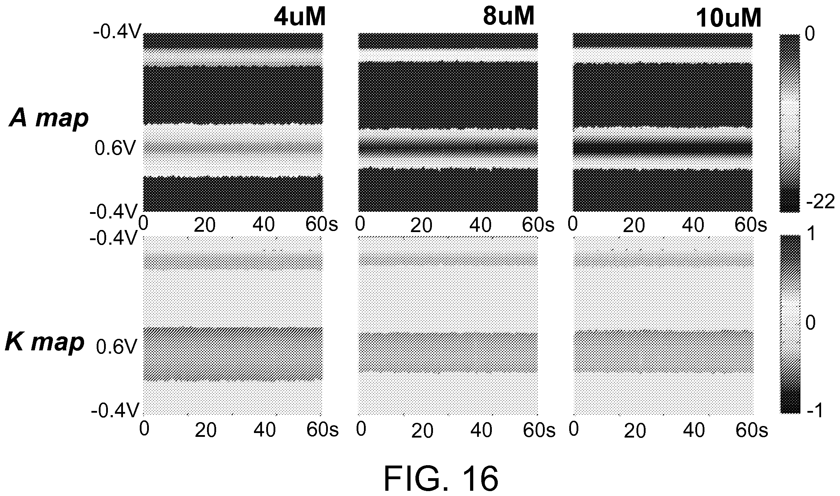

*K* maps and *A* maps can be obtained from KCV without background subtraction, such that absolute quantification of analytes is possible. An exemplary KCV waveform for DA + Tris buffer, without background subtraction, is shown in Figure 15. Figure 16 presents a series of images showing *A* maps and *K* maps for increasing concentrations of DA in Tris buffer, with kinetic information estimated from the original CV (without background subtraction).

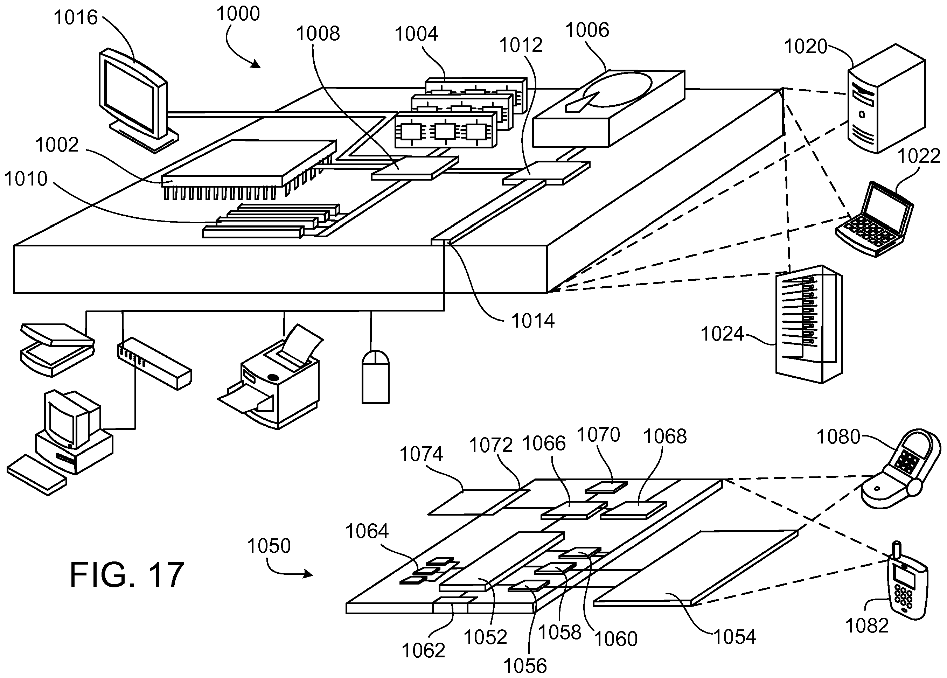

Figure 17 is a block diagram of computing devices 1000, 1050 that may be used to implement the systems and methods described herein, as either a client or as a server or plurality of servers. Computing device 1000 is intended to represent various forms of digital computers, such as laptops, desktops, workstations, personal digital assistants, servers, blade servers, mainframes, and other appropriate computers. Computing device 1050 is intended to represent various forms of mobile devices, such as personal digital assistants, cellular telephones, smartphones, and other similar computing devices. The components shown here, their connections and relationships, and their functions, are meant to be exemplary only, and are not meant to limit implementations described and/or claimed in this document.

Computing device 1000 includes a processor 1002, memory 1004, a storage device 1006, a high-speed interface 1008 connecting to memory 1004 and high-speed expansion ports 1010, and a low speed interface 1012 connecting to low speed bus 1014 and storage device 1006. Each of the components 1002, 1004, 1006, 1008, 1010, and 1012, are interconnected using various buses, and may be mounted on a common motherboard or in other manners as appropriate. The processor 1002 can process instructions for execution within the computing device 1000, including instructions stored in the memory 1004 or on the storage device 1006 to display graphical information for a graphical user interface on an external input/output device, such as display 1016 coupled to high speed interface 1008. In other implementations, multiple processors and/or multiple buses may be used, as appropriate, along with multiple memories and types of memory. Also, multiple computing devices 1000 may be connected, with each device providing portions of the necessary operations (e.g., as a server bank, a group of blade servers, or a multi-processor system).

The memory 1004 stores information within the computing device 1000. In one implementation, the memory 1004 is a volatile memory unit or units. In another implementation, the memory 1004 is a non-volatile memory unit or units. The memory 1004 may also be another form of computer-readable medium, such as a magnetic or optical disk.

The storage device 1006 is capable of providing mass storage for the computing device 1000. In one implementation, the storage device 1006 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described herein. The information carrier is a computer- or machine-readable medium, such as the memory 1004, the storage device 1006, or memory on processor 1002.

The high speed controller 1008 manages bandwidth-intensive operations for the computing device 1000, while the low speed controller 1012 manages lower bandwidth-intensive operations. Such allocation of functions is exemplary only. In one implementation, the high-speed controller 1008 is coupled to memory 1004, display 1016 (e.g., through a graphics processor or accelerator), and to high-speed expansion ports 1010, which may accept various expansion cards (not shown). In the implementation, low-speed controller 1012 is coupled to storage device 1006 and low-speed expansion port 1014. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 1000 may be implemented in a number of different forms, as shown in the figure. For example, it may be implemented as a standard server 1020, or multiple times in a group of such servers. It also may be implemented as part of a rack server system 1024. In addition, it may be implemented in a personal computer such as a laptop computer 1022. Alternatively, components from computing device 1000 may be combined with other components in a mobile device (not shown), such as device 1050. Each of such devices may contain one or more of computing device 1000, 1050, and an entire system may be made up of multiple computing devices 1000, 1050 communicating with each other.

Computing device 1050 includes a processor 1052, memory 1064, an input/output device such as a display 1054, a communication interface 1066, and a transceiver 1068, among other components. The device 1050 may also be provided with a storage device, such as a microdrive or other device, to provide additional storage. Each of the components 1050, 1052, 1064, 1054, 1066, and 1068, are interconnected using various buses, and several of the components may be mounted on a common motherboard or in other manners as appropriate.

The processor 1052 can execute instructions within the computing device 1050, including instructions stored in the memory 1064. The processor may be implemented as a chipset of chips that include separate and multiple analog and digital processors. Additionally, the processor may be implemented using any of a number of architectures. For example, the processor 410 may be a CISC (Complex Instruction Set Computers) processor, a RISC (Reduced Instruction Set Computer) processor, or a MISC (Minimal Instruction Set Computer) processor. The processor may provide, for example, for coordination of the other components of the device 1050, such as control of user interfaces, applications run by device 1050, and wireless communication by device 1050.

Processor 1052 may communicate with a user through control interface 1058 and display interface 1056 coupled to a display 1054. The display 1054 may be, for example, a TFT (Thin-Film-Transistor Liquid Crystal Display) display or an OLED (Organic Light Emitting Diode) display, or other appropriate display technology. The display interface 1056 may comprise appropriate circuitry for driving the display 1054 to present graphical and other information to a user. The control interface 1058 may receive commands from a user and convert them for submission to the processor 1052. In addition, an external interface 1062 may be provide in communication with processor 1052, so as to enable near area communication of device 1050 with other devices. External interface 1062 may provide, for example, for wired communication in some implementations, or for wireless communication in other implementations, and multiple interfaces may also be used.

The memory 1064 stores information within the computing device 1050. The memory 1064 can be implemented as one or more of a computer-readable medium or media, a volatile memory unit or units, or a non-volatile memory unit or units. Expansion memory 1074 may also be provided and connected to device 1050 through expansion interface 1072, which may include, for example, a SIMM (Single In Line Memory Module) card interface. Such expansion memory 1074 may provide extra storage space for device 1050, or may also store applications or other information for device 1050. Specifically, expansion memory 1074 may include instructions to carry out or supplement the processes described above, and may include secure information also. Thus, for example, expansion memory 1074 may be provide as a security module for device 1050, and may be programmed with instructions that permit secure use of device 1050. In addition, secure applications may be provided via the SIMM cards, along with additional information, such as placing identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described herein. The information carrier is a computer- or machine-readable medium, such as the memory 1064, expansion memory 1074, or memory on processor 1052 that may be received, for example, over transceiver 1068 or external interface 1062.

Device 1050 may communicate wirelessly through communication interface 1066, which may include digital signal processing circuitry where necessary. Communication interface 1066 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 1068. In addition, short-range communication may occur, such as using a Bluetooth, WiFi, or other such transceiver (not shown).

Device 1050 may also communicate audibly using audio codec 1060, which may receive spoken information from a user and convert it to usable digital information. Audio codec 1060 may likewise generate audible sound for a user, such as through a speaker, e.g., in a handset of device 1050. Such sound may include sound from voice telephone calls, may include recorded sound (e.g., voice messages) and may also include sound generated by applications operating on device 1050.

The computing device 1050 may be implemented in a number of different forms, as shown in the figure. For example, it may be implemented as a cellular telephone 1080. It may also be implemented as part of a smartphone 1082, personal digital assistant, or other similar mobile device.

Additionally computing device 1000 or 1050 can include Universal Serial Bus (USB) flash drives. The USB flash drives may store operating systems and other applications. The USB flash drives can include input/output components, such as a wireless transmitter or USB connector that may be inserted into a USB port of another computing device.

Various implementations of the systems and techniques described herein can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described herein can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described herein can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described herein), or any combination of such back end, middleware, or front end components. The components of the system can be interconnected by any form or medium of digital data communication (e.g., a communication network). Examples of communication networks include a local area network ("LAN"), a wide area network ("WAN"), peer-to-peer networks (having ad-hoc or static members), grid computing infrastructures, and the Internet.

The computing system can include clients and servers. A client and server are generally remote from each other and typically interact through a communication network. The relationship of client and server arises by virtue of computer programs running on the respective computers and having a client-server relationship to each other.

The invention will be further described in the following examples, which do not limit the scope of the invention described in the claims.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method for assessing a concentration of an analyte present within an environment, wherein said method comprises:
(a) obtaining, by a voltammetry device, voltammetric data by multi pulse cyclic voltammetry, with repeated series (100) of pulses (105) of a waveform,
(b) determining a voltammogram from said data,
(c) determining, by a computing system, a detection value based on said voltammogram, wherein said detection value provides an indication about said concentration, and
(d) providing information about said detection value or said concentration to a computer or for display to a user.

2. The method of claim 1, wherein said analyte is a chemical capable of being measured by electrochemistry.

3. The method of claim 1, wherein said environment is brain tissue *in vivo.*

4. The method of claim 1, wherein each of said series (100) of pulses (105) includes three to twenty pulses (105).

5. The method of claim 1, wherein each of said series (100) of pulses (105) is generated over about 1 millisecond to about 300 milliseconds.

6. The method of claim 1, wherein the repetition time (120) between the onset of consecutive series (100) of pulses (105) is between about 10 milliseconds and about 10 seconds.

7. The method of claim 1, wherein the lag time (130) between consecutive series (100) of pulses (105) is between about 1 millisecond and about 700 milliseconds.

8. The method of claim 1, wherein each pulse (105) lasts between about 0.15 millisecond and about 20 milliseconds.

9. The method of claim 1, wherein the delay time (150) between consecutive pulses (105) is between about 0.2 millisecond and about 20 milliseconds.

10. A computerized system for assessing a concentration of an analyte within a sample, comprising:
(a) a voltammetry device for obtaining voltammetric data by multi pulse cyclic voltammetry, with repeated series (100) of pulses (105) of a waveform,
(b) a communication port configured to receive said voltammetric data,
(c) one or more computer-readable storage media having recorded thereon instructions that, when executed, determines:
(i) a voltammogram from said data, and
(ii) a detection value based on said voltammogram, wherein said detection value provides an indication about said concentration, and
(d) a communication port configured to provide information about said detection value or said concentration.

11. A tangible, non-transitory computer program product comprising instructions that, when executed in a system in accordance with claim 10, causes said system to perform the method of claim 1.

12. The computer program product of claim 11, wherein said analyte is a chemical capable of being measured by electrochemistry.

## Patentansprüche

1. Verfahren zum Bewerten einer Konzentration eines Analyts, das in einer Umgebung vorhanden ist, wobei das Verfahren umfasst:
(a) Erhalten in einer Voltammetrievorrichtung von voltammetrischen Daten aus einer zyklischen Mehrpulscyclovoltammetrie mit wiederholten Serien (100) von Pulsen (105) einer Wellenform,
(b) Ermitteln eines Voltammogramms aus den Daten,
(c) Ermitteln durch ein Computersystem eines Erkennungswerts aufgrund des Voltammogramms, wobei der Erkennungswert eine Anzeige über die Konzentration bereitstellt, und
(d) Bereitstellen von Informationen über den Erkennungswert oder die Konzentration für einen Computer oder für eine Anzeige für einen Benutzer.

2. Verfahren nach Anspruch 1, wobei der Analyt eine Chemikalie ist, die mithilfe einer Elektrochemie messbar ist.

3. Verfahren nach Anspruch 1, wobei die Umgebung ein In-vivo-Gehirngewebe ist.

4. Verfahren nach Anspruch 1, wobei jede der Serien (100) von Pulsen (105) zwischen drei und zwanzig Pulse (105) enthält.

5. Verfahren nach Anspruch 1, wobei jede der Serien (100) von Pulsen (105) für eine Zeit zwischen ungefähr 1 Millisekunde und ungefähr 300 Millisekunden erzeugt wird.

6. Verfahren nach Anspruch 1, wobei die Wiederholungszeit (120) zwischen dem Beginn aufeinanderfolgender Serien (100) von Pulsen (105) zwischen ungefähr 10 Millisekunden und ungefähr 10 Sekunden liegt.

7. Verfahren nach Anspruch 1, wobei die Verzögerung (130) zwischen aufeinanderfolgenden Serien (100) von Pulsen (105) zwischen ungefähr 1 Millisekunde und ungefähr 700 Millisekunden liegt.

8. Verfahren nach Anspruch 1, wobei jeder Puls (105) zwischen ungefähr 0,15 Millisekunden und ungefähr 20 Millisekunden dauert.

9. Verfahren nach Anspruch 1, wobei die Verzögerungszeit (150) zwischen aufeinanderfolgenden Pulsen (105) zwischen ungefähr 0,2 Millisekunden und ungefähr 20 Millisekunden liegt.

10. Computergestütztes System zum Bewerten einer Konzentration eines Analyts in einer Probe, umfassend:
(a) eine Voltammetrievorrichtung zum Erhalten von voltammetrischen Daten aus einer zyklischen Mehrpulscyclovoltammetrie mit wiederholten Serien (100) von Pulsen (105) einer Wellenform,
(b) einen Kommunikationsanschluss, der ausgelegt ist zum Empfangen der voltammetrischen Daten,
(c) ein oder mehrere computerlesbare Speichermedien, in denen Befehle aufgezeichnet sind, die, wenn sie ausgeführt werden Folgendes ermitteln:
(i) ein Voltammogramm aus den Daten, und
(ii) einen Erkennungswert aufgrund des Voltammogramms, wobei der Erkennungswert eine Anzeige über die Konzentration bereitstellt, und
(d) einen Kommunikationsanschluss, der ausgelegt ist zum Bereitstellen von Informationen über den Erkennungswert oder die Konzentration.

11. Konkretes nichtflüchtiges Computerprogrammprodukt, das Befehle umfasst, die, wenn sie in einem System nach Anspruch 10 ausgeführt werden, das System veranlassen, das Verfahren nach Anspruch 1 auszuführen.

12. Computerprogrammprodukt nach Anspruch 11, wobei der Analyt eine Chemikalie ist, die mithilfe einer Elektrochemie messbar ist.

## Revendications

1. Procédé permettant d'évaluer une concentration d'un analyte présent dans un environnement, ledit procédé comprenant :
(a) l'obtention, par un dispositif voltamétrique, de données voltramétriques par voltamétrie cyclique à impulsions multiples, avec des séries (100) répétées d'impulsions (105) d'une forme d'onde,
(b) la détermination d'un voltamogramme à partir desdites données,
(c) la détermination, par un système informatique, d'une valeur de détection sur la base dudit voltamogramme, ladite valeur de détection fournissant une indication sur ladite concentration, et
(d) la fourniture d'informations sur ladite valeur de détection ou ladite concentration à un ordinateur ou pour présentation à un utilisateur.

2. Procédé selon la revendication 1, dans lequel ledit analyte est un produit chimique susceptible d'être mesuré par électrochimie.

3. Procédé selon la revendication 1, dans lequel ledit environnement est un tissu cérébral *in vivo.*

4. Procédé selon la revendication 1, dans lequel chacune desdites séries (100) d'impulsions (105) comprend trois à vingt impulsions (105).

5. Procédé selon la revendication 1, dans lequel chacune desdites séries (100) d'impulsions (105) est générée sur environ 1 milliseconde à environ 300 millisecondes.

6. Procédé selon la revendication 1, dans lequel le temps de répétition (120) entre le début de séries (100) consécutives d'impulsions (105) se situe entre environ 10 millisecondes et environ 10 secondes.

7. Procédé selon la revendication 1, dans lequel le temps de latence (130) entre des séries (100) consécutives d'impulsions (105) se situe entre environ 1 milliseconde et environ 700 millisecondes.

8. Procédé selon la revendication 1, dans lequel chaque impulsion (105) dure entre environ 0,15 milliseconde et environ 20 millisecondes.

9. Procédé selon la revendication 1, dans lequel le temps de retard (150) entre des impulsions (105) consécutives se situe entre environ 0,2 milliseconde et environ 20 millisecondes.

10. Système informatisé permettant d'évaluer une concentration d'un analyte dans un échantillon, comprenant :
(a) un dispositif voltamétrique permettant d'obtenir des données voltamétriques par voltamétrie cyclique à impulsions multiples, avec des séries (100) répétées d'impulsions (105) d'une forme d'onde,
(b) un port de communication conçu pour recevoir lesdites données voltamétriques,
(c) un ou plusieurs supports d'enregistrement lisibles par ordinateur sur lesquels sont enregistrées des instructions qui, lorsqu'elles sont exécutées, déterminent :
(i) un voltamogramme à partir desdites données, et
(ii) une valeur de détection sur la base dudit voltamogramme, ladite valeur de détection fournissant une indication sur ladite concentration, et
(d) un port de communication conçu pour fournir des informations sur ladite valeur de détection ou ladite concentration.

11. Produit-programme d'ordinateur tangible non transitoire comprenant des instructions qui, lorsqu'elles sont exécutées dans un système selon la revendication 10, amènent ledit système à exécuter le procédé selon la revendication 1.

12. Produit-programme d'ordinateur selon la revendication 11, dans lequel ledit analyte est un produit chimique susceptible d'être mesuré par électrochimie.
